# EUROPEAN PATENT SPECIFICATION

(11) **EP 2 272 589 B1**
(45) Date of publication and mention of the grant of the patent: **17.06.2015**
(21) Application number: 10178862.8
(22) Date of filing: 19.09.2003
(51) Int. Cl.: B01L 3/14, B01L 3/00, G01N 1/28

(54) **High bias gel tube and process for making tube**
Versuchsbehälter mit schrägem Gel und Verfahren zur Herstellung
Tube de gel hautement sollicite et procede de fabrication du tube

(30) Priority: 23.09.2002 US 412824 P
(43) Date of publication of application: 12.01.2011
(62) Divisional of application: 03754759.3
(73) Proprietor: Becton Dickinson and Company, Franklin Lakes, NJ 07417-1880 (US)
(72) Inventor: Manoussakis, Dimirios, Wyckoff, NJ 07481 (US); Bradshaw, Allen, Sumter, SC 29153 (US); Martin, Paul, Plymouth, PL17PW (GB)
(74) Representative: Von Kreisler Selting Werner - Partnerschaft von Patentanwälten und Rechtsanwälten mbB

(56) References cited:
- FR-A- 2 640 753
- US-A- 3 852 194
- US-A- 3 997 442
- US-A- 4 426 290
- US-B1- 6 238 578

## Description

### BACKGROUND OF THE INVENTION

### Field of the Invention

The invention relates to body fluid collection containers, in particular blood collection tubes, capable of separating phases of different density, using a gel separating medium.

### Discussion of the Related Art

Fluid collection tubes containing a thixotropic gel for separating phases of different densities, e.g., in blood, are well known. See, e.g., U.S. Patents Nos. 3,997,442, 4,257,886, 4,426,290, 4,770,779; and 6,238,578, the disclosures of which are hereby incorporated by reference. The gel is selected to have a density between that of the phases of blood which are to be separated. Upon centrifugation of a collected blood sample, the force of centrifugation forces the gel from a substantially non-flowing state to a more flowable state. In the flowable state, the gel migrates to a position between the two phases, e.g., between serum and clot portions. And upon cessation of centrifugation, the gel again becomes substantially non-flowable, thereby maintaining the separation between phases. Gel movement, i.e., getting adequate movement of the gel upon centrifugation, can sometimes be an issue. U.S. Patent No. 3,997,442 suggests one solution, but improvements are always desired.

### SUMMARY OF THE INVENTION

The invention relates to an improved fluid collection container, containing a gel separation medium. According to the invention, the gel is disposed in the tube in a manner and geometry that is readily manufacturable, and which overcomes potential gel movement issues which is defined in the subject-matter of claim 1.

### BRIEF DESCRIPTION OF THE DRAWINGS

Fig. 1 shows a tube containing a separator gel material according to an aspect of the invention.
Fig. 2 shows a cross-sectional view of a tube containing a separator gel material according to an aspect of the invention.
Fig. 3 shows a cross-sectional view of a tube containing a separator gel material according to an aspect of the invention.
Fig. 4 shows a cross-sectional view of a tube containing a separator gel material according to an aspect of the invention.
Fig. 5 shows a cross-sectional view of a tube containing a separator gel material according to an aspect of the invention.
Figs. 6A-6C show cross-section profiles for a tube containing a separator gel material according to an aspect of the invention.
Figs. 7A-7G show cross-sectional profiles for a tube containing a separator gel material according to an aspect of the invention.
Fig. 8 shows a cross-sectional view of a tube containing a separator gel material according to an aspect of the invention.

### DETAILED DESCRIPTION OF THE INVENTION

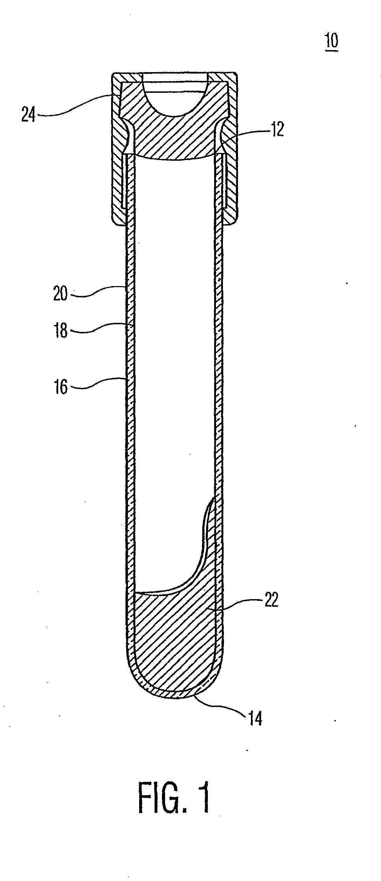

A typical blood collection tube according to the invention is shown in Fig. 1. The tube 10 contains an open upper end 12, a lower closed end 14, and sidewalls 16 having an inner wall 18 and an outer wall 20. A separating gel 22 is located within the container, at or adjacent the closed end 14.

The tube 10 is provided with a pierceable cap 24, that may be pierced by the non-patient end of a double ended blood collection needle. The tube 10 is generally evacuated, such that upon piercing by such a needle, blood is drawn into the tube. Details of evacuated blood collection tubes and blood collection are well known to those skilled in the art.

As noted above, upon sample collection, the tube is centrifuged to separate two phases of the blood sample, e.g., serum and red blood cells, or different cell types, as known in the art.

The invention provides the gel in the tube in an advantageous manner, that avoids or overcomes issues relating to gel movement.

According to the invention, a tube is provided with a gel separating material having an initial state that reflects an intermediate, transient state (during centrifugation) of a typical gel. In particular, the gel exhibits a state prior to any centrifugation that substantially resembles an intermediate state of an identical gel undergoing centrifugation in an identical container, wherein the initial state of the identical gel comprises an identical volume of the gel exhibiting a substantially planar exposed top surface. For example, where the exposed top surface of the identical gel exhibits a best-fit plane that exhibits an angle of 0 to 20° to a plane perpendicular to the longitudinal axis of the tube, the initial gel configuration of the invention would reflect an intermediate (during centrifugation) state of that identical gel.

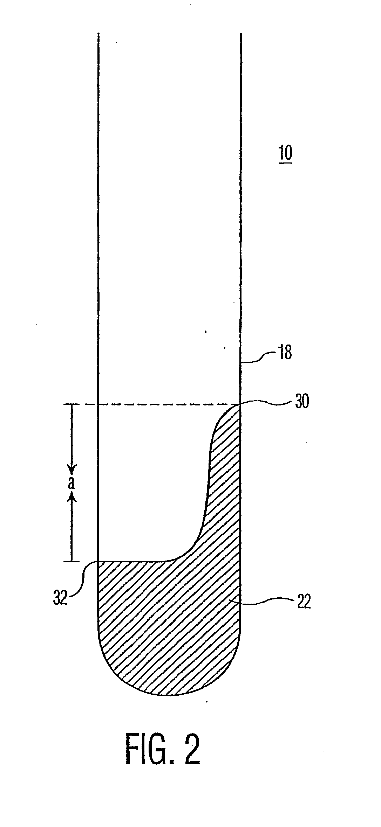

Embodiments of the gel location/geometry, from the outside of the tube, as well as some cross-section views, are shown in Figs. 2 to 9. It is possible to obtain the advantages of the invention by disposing the gel into the tube using a variety of principles and guidelines. The Figures show one type of design only, which is representative of the design guidelines presented herein. Variations based on the principles and description herein are also contemplated.

In one embodiment, reflected in Fig. 2, the distance a between the uppermost point 30 at which the gel 22 contacts the inner wall 18, and the highest point 32 at which the gel contacts the inner wall roughly opposite to the uppermost point, i.e., from 90° to 270° circumferentially, typically from 120° to 240°, most often including at least 180° circumferentially, is at least about 8 mm, typically about 8 to about 21 mm. Typically in this embodiment the gel, along a plane perpendicular to the longitudinal axis of the container and located halfway between the uppermost point and the highest point, exhibits less than 180° circumferential contact with the inner wall, typically less than 120°. (Circumferential contact indicates the extent to which the gel contacts the tube inner wall in a plane substantially perpendicular to the longitudinal axis of the tube.) Another way to describe this embodiment is that it is a configuration where, over 140 to 220° of circumferential contact, the gel exhibits a substantially uniform height in the container, relative to the lower end, and where the highest point at which gel contacts the inner wall of the container is about 8 to about 21 mm above the average height of the area having substantially uniform height.

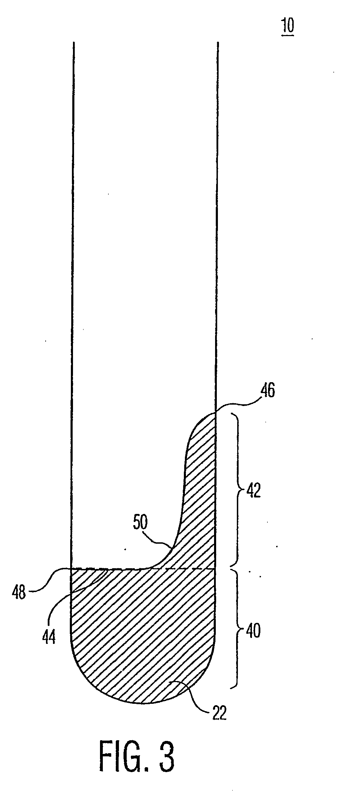

In another embodiment, reflected in Fig. 3, the gel comprises continuous first 40 and second 42 regions, the first region located at or adjacent to the closed lower end of the tube, and the second region extending upward from a portion of the first region.

Typically, the first region comprises an imaginary upper boundary 44 at which the first region exhibits 360° circumferential contact with the inner wall (typically 300 to 360° since some interruptions or regions without gel are possible in this planar upper boundary). The substantially planar upper boundary is typically defined as the surface having a best fit plane within 10° of a plane perpendicular to the longitudinal axis of the tube.

Typically, the uppermost point 46 of the second region is located at least about 8 mm higher than the uppermost point 48 of the upper boundary 44, more typically about 8 to about 21 mm.

Typically, the first region contains at least about 80 vol.% of the total gel, more typically at least about 90 vol.%, with a typical upper limit being about 95%.

The interior surface of the gel at the intersection 50 of the first and second regions is generally concave, and typically exhibits a radius of curvature of about 4 to about 8 mm. (The radius of curvature is defined as the radius of a best-fit sphere along that intersection.)

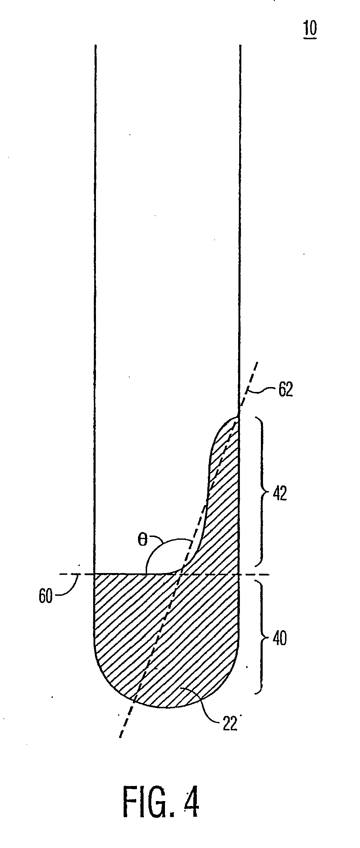

Typically, as reflected in Fig. 4, a best-fit plane 60 to the exposed surface of the first region facing the interior of the container exhibits an angle of 25° or less, more typically 10° or less, with a plane substantially perpendicular to the longitudinal axis of the container. The exposed surface of the second region facing the interior of the container defines a best-fit plane 62 exhibiting a 45 to 90° angle with a plane substantially perpendicular to the longitudinal axis of the container. (Best-fit plane indicates a plane that mathematically best fits the contour of the described surface or outline.)

Typically, the best fit plane to the exposed surface of the first region facing the interior of the container exhibits an angle θ of 90 to 140° with the best-fit plane to the surface of the second region facing the interior of the container.

Typically, along a plane perpendicular to the longitudinal axis of the container located halfway between the average height of the exposed surface of the first region and the uppermost point of the second region, the second region exhibits 80 to 140° circumferential contact with the inner surface.

Typically, the entirety of the second region exhibits less than 180° circumferential contact with the inner wall, generally less than 120°.

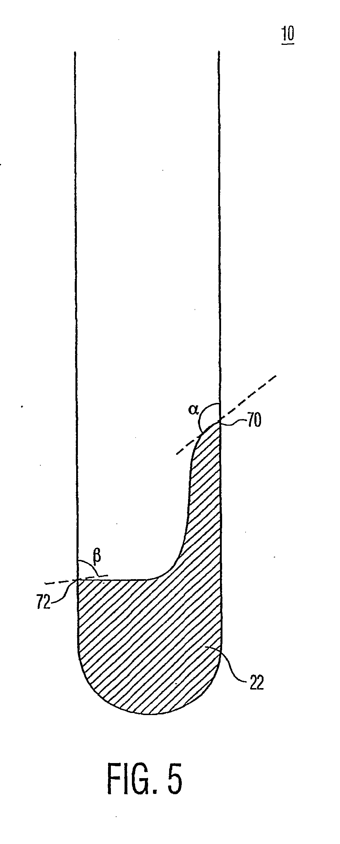

In a further embodiment, reflected in Fig. 5, at the uppermost point at which the gel contacts the inner wall, the angle between the inner wall and the tangent to the gel surface at the point of contact with the inner wall is about 100 to about 180°, and wherein, at the highest point at which the gel contacts the inner wall opposite the uppermost point, the angle between the inner wall and the tangent to the gel surface at the point of contact with the inner wall is about 70 to about 100°.

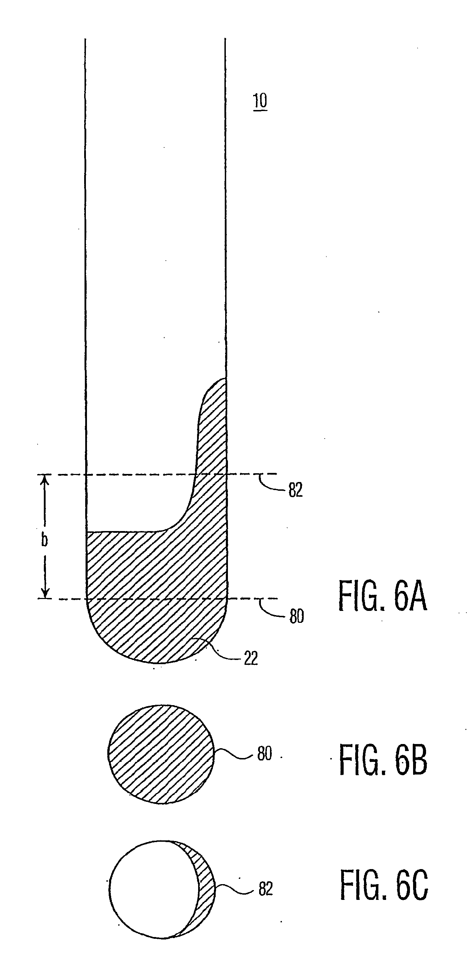

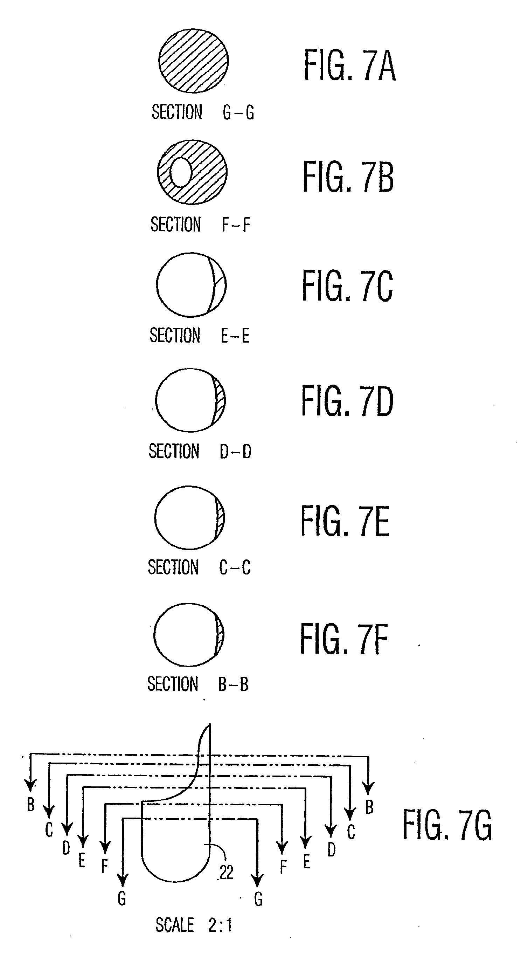

In another embodiment, reflected in Figs. 6A to 6C, upon superimposing on the gel first 80 and second 82 planes perpendicular to the longitudinal axis and spaced a distance b apart, the intersection between the first plane 80 and the gel defines a filled substantially circular or substantially elliptical shape, and the intersection between the gel and the second plane 82 defines a filled substantially crescent or substantially half-moon shape, such as shown in Fig. 6C, with b being a distance less than the distance between the uppermost point of gel contact with the tube inner wall and the bottom of the tube, and greater than the distance between the highest point of gel contact opposite the uppermost point and the bottom of the tube. Typical values for b are greater than 15 mm and less than 26 mm. An example of the cross-section of this embodiment at numerous locations is shown by Figs. 7A to 7G. In particular, Figs. 7A-7F shows the gel geometry at numerous cross-sections of the tube.

About 5 to about 20 vol.%, optionally about 10 to about 20 vol.%, of the gel is located within 8 to 12 mm of the uppermost point at which the gel contacts the inner wall.

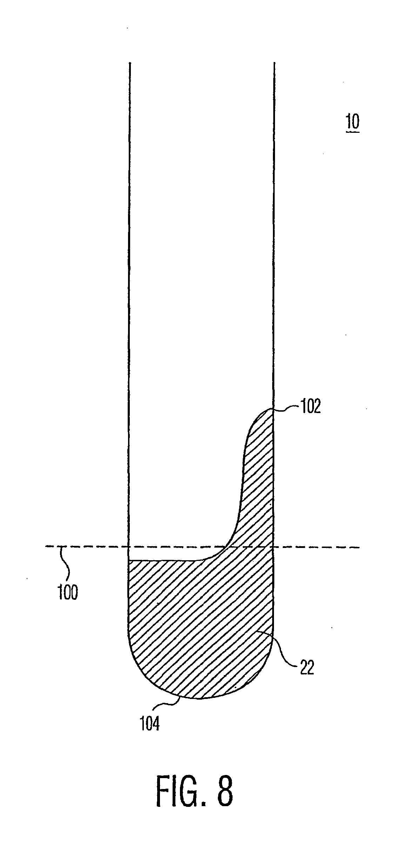

In a further embodiment, reflected in Fig. 8, about 10 to about 40 vol.%, more typically about 20 to about 40 vol.% of the gel 22 is located above a plane 100 perpendicular to the longitudinal axis and located halfway between the uppermost point 102 of the gel and the lowermost point 104 of the gel.

Common to all these embodiments is the advantage that they provide, both in gel movement and manufacturability. Gel movement is enhanced by the portion of the gel that extends toward the open end of the tube, e.g., the second region. Specifically, it is believed that providing such a gel extension toward the open end of the tube promotes initiation of gel movement at lower centrifugation speeds than would otherwise be required. The parameters for the gel geometry/placement herein provide for such a region that enhances gel movement upon centrifugation. Moreover, the geometry of the gel is readily attainable in manufacture, as discussed in more detail below.

A variety of separator gels, known in the art, are capable of being advantageously used in the invention. See, e.g., U.S. Patents Nos. 4,101,422, 4,148,764, and 4,350,593. In particular, acrylic-based, polyester-based, and hydrocarbon-based gels have all been found to be of use as separator materials, where such gels typically contain a resin modified with a particle such as fumed silica in order to form a networked gel.

Both plastic and glass tubes are possible. It is possible to dispose the gel into a tube by a variety of techniques. Generally, a nozzle capable of being inserted into the interior of the tube is used, with either the nozzle, the tubes, or both being moveable for that purpose. Dispensing of gel through the nozzle is normally initiated with the nozzle close to the desired location for the gel (to avoid putting gel on undesired regions of the tube), and as dispensing continues, the nozzle is then slowly drawn up the tube to avoid immersion in the gel. The gel is typically dispensed using pressure or other techniques known in the art. In addition, a tray of tubes is generally processed row by row to expedite manufacturing.

The desired geometry may be provided by various techniques. For example, it is possible to dispose gel into a tube using a nozzle, and then centrifuge the tubes at a particular angle and speed to provide the desired geometry. Such centrifuging may be done with an entire tray of tubes.

It is also possible to dispose the gel into a tube (or group of tubes) while holding the tube at an angle, or by angling the tubes during or after placing the gel into the tubes. The angle and gel deposition steps are controlled to provide the desired geometry. The tubes may then be left at ambient temperature, either at an angle or vertical. Some slumping of the gel may occur, such slumping taken into account when determining the steps necessary to reach the desired geometry.

It is also possible to use a nozzle having an opening oriented at an angle to the tube's axis. For example, the nozzle opening is positioned such that gel is disposed at an angle to the longitudinal axis, i.e., at an angle to vertical (more than one such off-axis nozzle opening is also possible). (There are a variety of techniques to configure a nozzle opening to dispose gel in this manner, including an opening at an angle to the axis of the nozzle, or an angled nozzle tip.) The angled nozzle opening is able to dispense gel in an asymmetric geometry in the tube. Useful angles for such an angled nozzle opening or angle nozzle tip are 25 to 45° to the longitudinal axis of the overall nozzle device, advantageously about 45°.

In some cases, it has been found that dispensing the gel under conditions (shear, temperature, viscosity, etc.) that allows the gel to slump from its initial dispensed position, to a final (prior to blood collection and centrifuge) position can be used advantageously to provide a desired geometry such as shown in the Figures. Such slumping may occur under ambient conditions post-dispensing, with the tube remaining in a vertical or angled position, e.g., the tube or tubes are simply moved to a location at which slumping and hardening are allowed to occur - no further actions (e.g., centrifuging) are required to obtain the advantageous geometry. If such slumping is desired, the gel may be dispensed in a manner that provides significant shear, such that the gel exhibits properties that allow such slumping. Conventionally, those in the art would seek to avoid such shear, to prevent such slumping after a dispensing step.

Specific conditions for the gel dispensing depends on, among other things, gel type, tube size, gel dispensing apparatus and techniques, and gel volume, as known to those skilled in the art.

Once the gel is allowed to slump and harden, the tube of the invention generally must go through additional processing steps. For examples, additives useful in blood or urine analysis, e.g., procoagulants or anticoagulants, may be disposed into the tube. As known in the art, blood analysis is often performed on serum, and procoagulants are typically used to enhance the rate of clotting. Such procoagulants include silica particles or enzyme clot activators such as elagic acid, fibrinogen and thrombin. If plasma is desired for analysis, an anticoagulant is generally used to inhibit coagulation, such that blood cells can be separated by centrifugation. Such anticoagulants include chelators such as oxalates, citrate, and EDTA, and enzymes such as heparin. Additives are disposed in the containers in any suitable manner, liquid or solid, including dissolution in a solvent, or disposing in powdered, crystallized, or lyophilized form.

Then, after any such additional additives are put into the tube, the tube (or group of tubes) is subjected to an evacuated chamber with a pressure below atmospheric pressure. A seal such as an elastomeric stopper or pierceable membrane is applied, and the tube is sterilized by a process such as irradiation (e.g., with cobalt 60 radiation), ethylene oxide gas exposure, or electron-beam exposure. (Note that several of these steps may be performed in an order other than that presented above).

The containers of the invention are capable of being formed in any desired size. Standard blood collection tubes with outside diameters of 13 x 75 mm or 16 x 100 mm are used.

Other embodiments of the invention will be apparent to those skilled in the art from consideration of the specification and practice of the invention disclosed herein. For example, while the gel geometry of the above embodiments reflects a single region that sweeps upward from a larger region of gel, it is possible to have more than one region sweeping upward, or to have one or more thin regions of gel (e.g., beads of gel) sweep upward.

## Claims

1. An evacuated tube (10) having outside dimensions of 13 x 75 mm or 16 x 100 mm comprising:
an upper end (12), a lower end (14), a sidewall (16) between the upper and lower ends (12, 14) having inner and outer walls (18, 20) and a pierceable closure (24); and
a gel (22) located in the container (10) in contact with a portion of the inner wall (18), **characterized in that**
5 to 20 vol.% of the gel (22) is located within 8 to 12 mm of the uppermost point at which the gel (22) contacts the inner wall (18).

2. The tube (10) of claim 1, wherein 10 to 20 vol.% of the gel (22) is located within 8 to 12 mm of the uppermost point at which the gel (22) contacts the inner wall (18).

3. The tube (10) of claim 1, wherein the gel (22) is a thixotropic gel.

4. The tube (10) of claim 1, wherein the lower end (14) is closed, and wherein the gel (22) is disposed at the closed lower end (14).

## Patentansprüche

1. Evakuiertes Röhrchen (10) mit Außenabmessungen von 13 x 75 mm oder 16 x 100 mm, umfassend:
ein oberes Ende (12), ein unteres Ende (14), eine Seitenwand (16) zwischen dem oberen und dem unteren Ende (12, 14), die eine Innen- und eine Außenwand (18, 20) aufweist, und einen durchstechbaren Verschluss (24); und
ein Gel (22), das sich in dem Behälter (10) in Kontakt mit einem Teil der Innenwand (18) befindet, **dadurch gekennzeichnet, dass**
sich 5 bis 20 Vol.-% des Gels (22) innerhalb von 8 bis 12 mm vom obersten Punkt, an dem das Gel (22) mit der Innenwand (18) in Kontakt steht, befinden.

2. Röhrchen (10) gemäß Anspruch 1, wobei sich 10 bis 20 Vol.-% des Gels (22) innerhalb von 8 bis 12 mm vom obersten Punkt, an dem das Gel (22) mit der Innenwand (18) in Kontakt steht, befinden.

3. Röhrchen (10) gemäß Anspruch 1, wobei das Gel (22) ein thixotropes Gel ist.

4. Röhrchen (10) gemäß Anspruch 1, wobei das untere Ende (14) geschlossen ist und wobei sich das Gel (22) am geschlossenen unteren Ende (14) befindet.

## Revendications

1. Tube sous vide (10) ayant des dimensions extérieures de 13 x 75 mm ou 16 x 100 mm comprenant :
une extrémité supérieure (12), une extrémité inférieure (14), une paroi latérale (16) entre les extrémités supérieure et inférieure (12, 14) ayant des parois intérieure et extérieure (18, 20) et une fermeture perforable (24) ; et
un gel (22) situé dans le récipient (10) en contact avec une partie de la paroi intérieure (18), **caractérisé en ce que**
5 à 20% en volume du gel (22) sont situés à 8-12 mm du point le plus élevé au niveau duquel le gel (22) entre en contact avec la paroi intérieure (18).

2. Tube (10) de la revendication 1, dans lequel 10 à 20% en volume du gel (22) sont situés à 8-12 mm du point le plus élevé au niveau duquel le gel (22) entre en contact avec la paroi intérieure (18).

3. Tube (10) de la revendication 1, dans lequel le gel (22) est un gel thixotrope.

4. Tube (10) de la revendication 1, dans lequel l'extrémité inférieure (14) est fermée, et dans lequel le gel (22) est disposé au niveau de l'extrémité inférieure fermée (14).
